# EUROPEAN PATENT APPLICATION

(11) **EP 1 118 603 A2**
(43) Date of publication of application: **25.07.2001**
(21) Application number: 01500009.4
(22) Date of filing: 12.01.2001
(51) Int. Cl.: C05F 9/04, C05F 17/00

(54) **Process for the transformation of organic remains into organic fertilizer and by-products and worm breeding**

(30) Priority: 18.01.2000 ES 200000096
(71) Applicant: Maimo Crespi, Pere, 07620 Lluchmajor (ES); Huguet Rojas, Juan Gabriel, 07620 Lluchmajor (ES)
(72) Inventor: Maimo Crespi, Pere, 07620 Lluchmajor (ES); Huguet Rojas, Juan Gabriel, 07620 Lluchmajor (ES)
(74) Representative: Marques Alos, Fernando

(57) **Abstract**

**"PROCESS FOR THE TRANSFORMATION OF ORGANIC REMAINS INTO ORGANIC FERTILIZER AND BY-PRODUCTS AND WORM BREEDING"**, first we set up a selection of different sources of organic stuff to impoverish, which is triturated, if it was necessary, to condition the product to its suitable size and mix to get a fixed protein level among different remains and residues, afterwards, the mixture is ventilated and it is left to settle during a period of about three or six months. We must control different parameters such as pH, humidity, temperature and chemical composition. After some time of rest we introduce it into a deposit, cradle or hopper and we add the worms, extending on the group translucent material and approximately six months later we separate the humus or worm compost formed, solid or liquid, which is taken to warehouses to mature, later it will be packed and commercialized.

## Description

### DESCRIPTION OF THE INVENTION

We express the object of the present invention in the descriptive memory as **"PROCESS FOR THE TRANSFORMATION OF ORGANIC REMAINS INTO ORGANIC FERTILIZER AND BY-PRODUCTS AND WORM BREEDING"**, applied to recycling of the organic remains the production of organic fertilizer and reproduction of impoverishing worms of the organic material for it sale.

Nowadays, the recycling of industrial or urban solid remains is having a period of substantial prosperity.

Indeed it is due to the fact that awakening of the people about the problem, we generate an excess of consume products, due to the modernization and industrialization of the present society.

As a result, the quantity of remains produced is bigger and consequently their treatment in order to minimize or reuse them is urgent.

Nowadays, the organic material may be treated in industrial plants, where it suffers an impoverishment in a carbonate sequence to get useful compounds as fertilizers.

However, in the industrial plants the fertilizer that we get is of low quality and the commercialization is quite difficult, even at low prices, since in its elaboration only an impoverished bacterial process operates.

As a result, we have developed a new transformation process of the organic wastes into organic fertilizer and worm breeding.

Basically, the new process starts from a selection of different organic material sources to impoverish.

That organic material is ground to arrange the product to the adequate size, if it is necessary, and blended in such a way that among the different remains and wastes one obtains a certain protein level among them.

With this aim in view we will arrange a piece of land with a smooth slope towards a point in which one excavates a hole or a pit with the adequate size to retain slimes that later we will use to keep the mixture at its necessary humidity level.

Immediately after, a plastic material is spread out on that piece of land to stop the loss of the mixture of the liquids flowing towards the surface. Next, to avoid the perforation, of the material a wooden board is placed on it.

The mixture must be ventilated, as the bacteria act in an aerobic atmosphere.

The mixture is ventilated and it is left to settle during a period of about three or six months, we must control different parameters such as pH; humidity, temperature and the chemical composition.

The mixture after the above mentioned period is introduced into a cradle or hopper.

To build this cradle we delimitate the plot perimeter to use with stakes previously treated to avoid decomposition. On these stakes a 6-mm. thick geotextile fabric is nailed down.

With regard to the cradle, one must protect its building and materials used. The delimitation of contention can be made of wood, brick, metal or any other suitable material and its construction can be carried out in places where it does not rain a lot, and because of this one must dig a ditch on the ground to contain the product and avoid the worms escape.

On the other hand, we have to care for the cradle position, which can be put up outdoors, in a cellar, caves, industrial plants, greenhouses ...etc.

Once the mixture is placed, we add the worms and on the cradle we put a translucent material to avoid water evaporation, the inclemency of the weather and predator.

Instead of a cradle, a hopper can be employed. This hopper consists of a cylinder - of metal, plastic or any other suitable material-equipped with a trap door through which the humus or the worm excrements can be taken out with a metallic grating or any other suitable material, which function will be to protect the worms from predators. The trap door can be obviated, taking the content out from above.

Whether if we choose a deposit, a hopper or a cradle, the organic material to impoverish has to be supervised by the same parameters aforementioned. Likewise, we will add organic material, as the worms impoverish and convert it into humus, or worm compost and its by-products.

At the end of approximately six months, we separate the humus produced and put it into store to mature and being later packed and commercialized .

The process itself can produce and excess of worms, which can be packed to be sold as food for fish-farms, flour for human or animal feed, pharmaceutical applications, as fishing baits, etc.

Worm live in a wet background, and feed on organic vegetable rests, as well as animals in decomposition.

Their main virtue is that they do not transmit sicknesses.

The type of worm that we deal with belongs to the "epigaios" family, which lives on the surface and feeds from organic material, producing humus.

This new process is suitable for further use of organic waste coming from different origins, such as animal manure, wood remains (sawdust, shavings), scraps from meat industries and slaughterhouses, forest wastes, mud from purifying plants, papers, pasteboard, ashes, etc.

The humus obtained in this brand new process can be employed as fertilizer in agriculture.

Likewise, the environment created inside the cradle or the hopper is favourable to generate an excess of worms, suitable for further sale.

The worms can be used for the production of flour for animals and human feed, as fishing baits, pharmaceutical applications, food for fish farms and for carnivorous and omnivorous animals, etc.

The main advantage of the new method lies in obtaining a perfect fertilizer from the impoverishment of organic material, which nowadays is an ecological problem, by the worms. As the same time, we can create a favourable environment for the breeding of worms.

### DESCRIPTION OF THE DRAWINGS

With the aim of illustrating what we have been previously stated, we include to this descriptive memorandum, and being an integral part of it, a sheet with drawings where we show in a simplified and schematic way, an example only illustrative but not restrictive, of the practical possibilities of this invention.

In these drawings figure-1 correspond to a scheme of the new process for the transformation of organic residues into organic fertilizer, and other by-products, as well as worm breeding.

### DESCRIPTION OF A PRACTICAL CASE

The new process for the transformation of organic remains into organic fertilizer and by-products as well as worm breeding starts from a selection (1) of the different sources of organic material to impoverish. That organic material is ground (2), if it is necessary, to arrange the product to the adequate size, , and blended (3) in such a way that among the different remains and wastes.

For that purpose we will arrange a piece of land with a smooth slope toward a point in which one excavates a hole or a pit with the adequate size to retain slimes that later we will use to keep the necessary humidity level of the mixture.

Immediately after, a plastic material is spread out in that piece of land to stop the loss of liquid of the mixture towards the surface. Next, to avoid the perforation of the material a board support is placed on it.

The mixture must be ventilated, as the bacteria act in an aerobic atmosphere.

The mixture is ventilated and it is left to settle during a period of about three or six months, we must control different parameters such as pH, humidity, temperature and chemical composition.

The mixture must be ventilated, as the bacteria act in an aerobic atmosphere.

The mixture is ventilated and it is left to settle during a period of about three or six months, we must control different parameters such as pH; humidity, temperature and the chemical composition.

The mixture, after the above-mentioned period, is introduced into a cradle or hopper.

To build this cradle we delimitate the perimeter of the plot to use with stakes previously treated to avoid decomposition. On these stakes a 6-mm. thick geotextile fabric is nailed down.
With regard to the cradle, one must attend its building and materials used, the control delimitation can be made of wood, brick, metal or any other suitable material and its construction can be carried out in places where it does not rain a lot, and because of this one must dig a ditch on the surface to contain the product and avoid that the worms can escape.

Once placed the mixture, we add the worms (6) and on the cradle we put a translucent material to avoid water evaporation, the inclemency of the weather and the predator's voracity.

Instead of a cradle, a hopper can be employed, this hopper consists of a cylinder- of metal, plastic or any other suitable material-equipped with a trap door through which the humus or the worm excrements can be taken out, with a metallic grating or any other suitable material which function will be to protect the worms from predators, the trap door can be obviated, taking the content out from above.

Whether if we choose a deposit, a hopper or a cradle , the organic material to impoverish has to be supervised by the same parameters previously mentioned.

Likewise, we shall add organic material as the worms impoverish and convert it into humus or worm compost and its derivatives.

At the end of approximately six months we separate the humus produced (7) and put it into store for its maturing (8), and further packaging and commercialization (9).

The process itself can produce an excess of worms (10) which can be packed to be sold later (11).

Once stated the concept we write the following claims, summing up the new techniques that we want to claim:

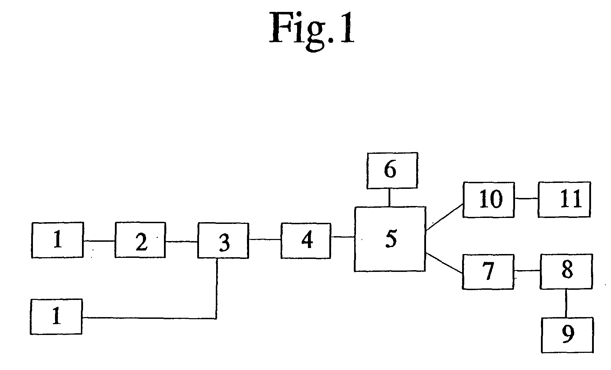

## Claims

1. **"PROCESS FOR THE TRANSFORMATION OF ORGANIC REMAINS INTO ORGANIC FERTILIZER AND BY-PRODUCTS AND WORM BREEDING"**, essentially distinguished because part of the selection to different sources of organic stuff to impoverish, which is triturated, if it was necessary, to condition the product to its adequate size and mixture to get a fixed protein level among different remains and residues, subsequently the mixture is ventilated and it is left to settle during a period of about three or six months, we must control different parameters such as pH, humidity, temperature and chemical composition, after some time of rest, we introduce it into a deposit, cradle o hopper.

2. **"PROCESS FOR THE TRANSFORMATION OF ORGANIC REMAINS INTO ORGANICS FERTILIZER AND BY-PRODUCTS AND WORM BREEDING"**, according to the preceding claim distinguished because with this aim in view we will arrange a piece of land equipped with a smooth slope towards a point in which one excavates a hole or a pit with the adequate size to retain leach that further we will use to keep the necessary humidity level of the mixture. Immediately after, a plastic material is spread out on that piece of land to stop the loss of the liquids towards the surface. Then to avoid perforation of the material a board of wood is placed on it.

3. **"PROCESS FOR THE TRANSFORMATION OF ORGANIC REMAINS INTO ORGANIC FERTILIZER AND BY-PRODUCTS AND WORM BREEDING"** according to preceding claims distinguished because once placed the mixture in the deposit or cradle, we add the worms and over the cradle we put a translucent material to avoid water evaporation, the harshness of the weather and the voracity of predators.

4. **"PROCESS FOR THE TRANFORMATION OF ORGANIC REMAINS INTO ORGANIC FERTILITZER AND BY-PRODUCTS AND WORM BREEDING"** according to preceding claims distinguished because building this cradle, we delimitate the perimeter of the plot to use with stakes, which have been previously treated to avoid decomposition, on these stakes a 6-mm. thick geotextile fabric is nailed down.

5. **"PROCESS FOR THE TRANSFORMATION OF ORGANIC REMAINS** **INTO ORGANIC FERTILIZER AND BY-PRODUCTS AND WORM BREEDING"** according to preceding claims distinguished because instead of a cradle, a hopper can be employed this hopper consist of a cylinder - of metal, plastic or any other suitable material- equipped with a trap door for the worms defecation or humus exit, and with a metallic grating on top to protect the worms from predators.

6. **"PROCESS FOR THE TRANSFORMATION OF ORGANIC REMAINS INTO ORGANIC FERTILIZER AND BY-PRODUCTS AND WORM BREEDING"** according to preceding claims distinguished because the trap door can be obviated, taking the content out from the top.

7. **"PROCESS FOR THE TRANSFORMATION OF ORGANIC REMAINS INTO ORGANIC FERTILIZER AND BY-PRODUCTS AND WORM BREEDING"** according to preceding claims distinguished because in the cradle or hopper we add organic material to impoverish and converted into humus, compost worms and by-products.

8. **"PROCESS FOR THE TRANSFORMATION OR ORGANIC REMAINS INTO ORGANIC FERTILIZER AND BY-PRODUCTS AND WORM BREEDING"** according to preceding claims distinguished because approximately after six months we separate the humus or compost worms formed, solid or liquid, get ready to put in a warehouse to mature, later it will be packed and commercialized as worm compost, liquid humus or fertilizer enriched with humus or any other possible by-products.

9. **"PROCESS FOR THE TRANSFORMATION OF ORGANIC REMAINS INTO ORGANIC FERTILIZER AND BY-PRODUCTS AND WORM BREEDING"** according to preceding claims distinguished because the environment into cradles or hoppers is propitious to produce an excess of worms, enough to be packed and commercialized.
